# EUROPEAN PATENT APPLICATION

(11) **EP 3 854 453 A1**
(43) Date of publication of application: **28.07.2021**
(21) Application number: 20153569.7
(22) Date of filing: 24.01.2020
(51) Int. Cl.: A61N 5/10, C12M 3/00, C12N 5/00, C12M 1/00, G01T 1/11, G01T 1/02

(54) **FLASH DETECTOR**

(71) Applicant: CHUV, Centre hospitalier universitaire vaudois, 1011 Lausanne, Vaud (CH)
(72) Inventor: Buchillier, Thierry, 1010 Lausanne (CH); Bailat, Claude, 1806 St Légier (CH); Vozenin, Marie-Catherine, 1163 Etoy (CH)
(74) Representative: Vossius & Partner Patentanwälte Rechtsanwälte mbB

(57) **Abstract**

The present invention relates to a kit for evaluating FLASH capability of a radiation beam, the kit including a biological dosimeter comprising a test chamber containing fertilized non-mammalian eggs, wherein the test chamber is further configured to receive a beam of radiation; and a physical dosimeter comprising at least one type of a radiation dosimeter. The invention further relates to a method for evaluating FLASH capability of a radiation beam utilizing a biological dosimeter and a physical dosimeter.

## Description

### Background

Radiation therapy is a cornerstone of cancer treatment and is used in over 50% of cancer patients, however its efficacy remains suboptimal in many radiation-resistant tumors. Although recent development in radiotherapy have allowed for higher precision and efficacy, the collateral damages to healthy tissue remain a limitation and being able to deliver high curative radiation doses to tumors depends on the ability to spare normal tissues from harmful effects of radiation. Today this goal is now at hand using FLASH-radiotherapy (FLASH-RT). FLASH-RT consists of dose delivery within a comparitively short irradiation time, for example, the radiation dose may be delivered in milliseconds whereas with radiotherapy at a conventional dose rate the dose may be delivered over minutes. The main interest of FLASH-RT is its biological effect, namely, impressive sparing of normal tissue at doses enabling remarkable tumor control. This biological effect has been called the FLASH effect (review in Vozenin MC, Hendry JH, Limoli CL. Biological Benefits of Ultra-high Dose Rate FLASH Radiotherapy: Sleeping Beauty Awoken. Clin Oncol (R Coll Radiol). 2019 Jul;31(7):407-415. doi: 10.1016/j.clon.2019.04.001. Epub 2019 Apr 19. PubMed PMID: 31010708).

The main challenge for the clinical transfer of FLASH-RT relies upon the development of radiation beams able to deliver the high dose rate of irradiation able to produce the biological FLASH effect. In order to develop such beams, having access to a quick and reliable method for evaluating the FLASH capability of any radiation beam is highly advantageous.

The object of the invention is to provide a kit and a method for evaluation of the FLASH capability of a radiation beam.

### Summary

This object is achieved with the features of the independent claims. Dependent claims refer to preferred aspects of the invention.

According to a first aspect, the invention relates to a kit for evaluating FLASH capability of a radiation beam, the kit including a biological dosimeter comprising a test chamber containing fertilized non-mammalian eggs, wherein the test chamber is further configured to receive a beam of radiation; and a physical dosimeter comprising at least one type of a radiation dosimeter. The combination of physical and biological dosimeters allows an end user to evaluate the total radiation dose delivered and whether the potential protective effects (FLASH effect) produced by the tested beam have been achieved. Providing both in combination as a kit allows for ease of use and reliable, reproducible FLASH-RT results for developers of radiation beams.

Preferably the fertilized eggs are selected from zebrafish eggs, xenopus eggs, and/or drosophila eggs. Each of these egg types are well-studied and validated as FLASH-sensitive and thus can be easily evaluated for any abnormal development due to radiation exposure (see Montay-Gruel, Pierre, et al. "Long-term neurocognitive benefits of FLASH radiotherapy driven by reduced reactive oxygen species" PNAS, May 2019, 116(22) 10943-10951; and E. Beyreuther et al., Feasibility of proton FLASH effect tested by zebrafish embryo irradiation. Radiother Oncol 139, 46-50 (2019)). If the eggs are zebrafish eggs, it is preferred that they are provided between 0.5 hours and 36 hours after fertilization, preferably between 1 hour and 30 hours after fertilization, more preferably between 4 hours and 24 hours after fertilization. In these particular time ranges the zebrafish embryos are more sensitive to radiation effects.

Preferably the fertilized eggs comprise at least 5 eggs, preferably at least 10 eggs, more preferably at least 15 eggs, most preferably at least 20 eggs. A certain number of eggs aids in providing sufficient statistics for morphological evaluation.

Preferably the at least one radiation dosimeter is a passive dosimeter, preferably wherein the physical dosimeter comprises at least one of the following dosimeter types: an alanine detector, a Gafchromic film, and a thermoluminescent detector. The physical dosimeter may also comprise three different types of radiation dosimeters, preferably wherein the physical dosimeter comprises at least one of an alanine detector, a Gafchromic film, and a thermoluminescent detector. By providing multiple detectors the absorbed radiation dose can be measured with more accuracy.

Preferably the kit comprises a test chamber within which both the biological dosimeter and the physical dosimeter are located. By combining the biological dosimeter and the physical dosimeter within a single test chamber, the end user is provided with a convenient, easy manipulable device for FLASH determination.

Preferably the test chamber has a first dimension of between 2 and 10 cm, a second dimension of between 1 and 5 cm, and a third dimension of between 1 and 5 cm adaptable to beam energy and characteristics. Preferably the test chamber comprises a translucent substrate with a central cavity, wherein the physical dosimeter and the biological dosimeter are located within the cavity.

Preferably the biological dosimeter further comprises a medium within the test chamber, the medium being adapted to promote survival of the fertilized eggs, preferably wherein the medium has a volume of at least 200 µL, more preferably at least 500 µL, and even more preferably at least 1 mL. Preferably the medium comprises water comprising between 10 mg/L and 100 mg/L of ocean salt, preferably between 40 mg/L and 80 mg/L ocean salt, more preferably between 50 mg/L and 70 mg/L ocean salt, most preferably 60 mg/L ocean salt. Said medium is favorably adapted for the survival of saltwater fish embryos, such as zebrafish embryos.

According to a second aspect, the invention relates to a method for evaluating FLASH capability of a radiation beam, the method comprising the steps of:
(i) providing a biological dosimeter comprising a test chamber and fertilized non-mammalian eggs positioned within the test chamber;
(ii) providing a physical dosimeter comprising at least one type of radiation dosimeter;
(iii) irradiating the biological dosimeter with a radiation beam having a first configuration;
(iv) irradiating the physical dosimeter with a radiation beam having the first configuration;
(v) incubating the irradiated eggs for a predetermined time;
(vi) evaluating one or more morphology features of the fertilized eggs after incubation;
(vii) evaluating the irradiated physical dosimeter; and
(viii) classifying the FLASH capability of the radiation beam based at least in part on the evaluation of the irradiated physical dosimeter and the morphology evaluation.

This method for evaluating FLASH capability pairs the measured absorbed radiation dosage of the physical dosimeter with the evaluated effects on the biological dosimeter such that a provider of radiotherapy can reliably determine whether FLASH radiation is being provided.

Preferably evaluating the morphology features of the fertilized eggs comprises generating a summary morphology statistic derived from measurements made of each incubated fertilized egg, preferably by measuring a greatest cross-sectional length of each of the embryos and/or survival evaluation of the eggs. A summary morphology statistic incorporates data from each of the embryos wherein the cross-section length of the embryo is an indicator of healthy development. Embryo survival provides a count of how many embryos have survived irradiation.

Preferably evaluating the morphology comprises generating a summary morphology statistic derived from measuring a length of the incubated fertilized eggs, measuring a diameter of the eyes, measuring a size of the head, determining the degree of curvature of the organism, and/or evaluating the survival of the eggs. Depending on the type of fertilized eggs being used, different morphological parameters may be the best indicators of normal development.

Preferably evaluating the physical dosimeter radiation measurement comprises reading at least one radiation dosimeter and deriving an absorbed dose to water value or evaluating the physical dosimeter radiation measurement may comprise reading at least two dosimeters, preferably at least three dosimeters, and generating an absorbed dose to water value, wherein each dosimeter operates based on a different physical radiation measurement mechanism. A physical dosimeter supplies an objective measure of the total amount of radiation imparted by a radiotherapy treatment configuration. In some cases multiple dosimeters may provide a higher degree of reliability of the total radiation dose measurement.

Preferably classifying the FLASH capability of the first configuration based on the evaluation of the irradiated physical dosimeter and the morphology evaluation comprises determining whether the summary morphology statistic meets a predetermined threshold; and determining whether the summary physical dosimeter statistic meets a predetermined threshold.

Preferably the predetermined threshold for the summary morphology statistic is at most a 20% reduction from a summary morphology statistic derived from non-irradiated fertilized eggs of the same developmental age, preferably at most a 15% reduction, more preferably at most a 10% reduction. Such thresholds for the morphology statistic are determined as embryos damaged by radiation often fail to develop to full length.

Preferably the method further comprises repeating the steps i) through vii) using a second configuration of the radiation beam, and wherein classifying the FLASH capability of the radiation beam comprises the evaluation of the irradiated physical dosimeter and performing the morphology evaluation of the first and second configurations. In this configuration the FLASH radiation configuration can be compared against a traditional radiation configuration to establish whether FLASH radiation has been achieved.

Preferably the second configuration of the radiation beam is configured to provide the same radiation dose over a timescale at least 100 times longer than the first configuration, and wherein the predetermined threshold for the summary morphology statistic is at least a 10% increase from a summary morphology statistic derived from the second configuration of the radiation beam, preferably at least a 15% increase, more preferably at least a 20% increase. Such a threshold exhibits the pitfalls of traditional radiation schemes which result in damage to healthy tissues, in comparison with the protective effects of FLASH-RT. It may also be advantageous if the first predetermined threshold for the value of the absorbed dose to water measured in Gy, which when applied to fertilized eggs over a timescale at least 100 times longer than a total duration of the first configuration results in shortening of the average fertilized egg length after incubation relative to normal fertilized egg development by at least 3%, preferably at least 5%, more preferably at least 7%.

Preferably incubation comprises maintaining the irradiated eggs at a temperature between 20°C and 35°C, preferably between 25°C and 30°C, more preferably between 25°C and 29°C, most preferably at 28°C. Said incubation temperatures are common for simple organisms, such as zebrafish, xenopus, and drosophila. The predetermined time for incubation may be between 1 and 10 days, preferably between 2 and 5 days, more preferably between 3 and 5 days. In this time span each of the non-mammalian embryos can develop markedly in size and structural features.

Preferably the method comprises the further step of stopping the development of the fertilized eggs after incubation, more preferably by immersing the fertilized eggs in a tricaine solution; and/or fixing the fertilized eggs, preferably by immersing them in a solution comprising PFA and PBS. This helps to provide a standard end point for the development and a conformity of morphological evaluation.

Preferably steps iii) and iv) of the method are performed simultaneously. This helps to ensure that the same radiation configuration is provided to both the biological dosimeter and the physical dosimeter.

Preferably each dosimeter is chosen from the list comprising: an alanine dosimeter, Electron paramagnetic resonance (EPR) dosimeter, a Gafchromic film dosimeter, a thermoluminescent dosimeter, and a radiochromic film dosimeter. Each of these dosimeters provides certain advantages and drawbacks, including specific sensitivity ranges and saturation points.

Preferably the step of providing a biological dosimeter comprises breeding non-mammalian eggs; selecting the non-mammalian eggs that are fertilized; and positioning the fertilized non-human eggs into the test chamber.

### Brief Description of the Drawings

The invention will be explained in more detail in the following text with reference to preferred exemplary embodiments which are illustrated in the appended drawings, in which:
Fig. 1 schematically shows a configuration of a kit for evaluating FLASH capability of a radiation beam;
Fig. 2 schematically shows an exemplary configuration of a kit for evaluating FLASH capability of a radiation beam;
Fig. 3 provides an example of zebrafish embryo which has either developed normally (control), been exposed to radiation without FLASH effect, or been exposed to radiation with FLASH effect;
Fig. 4 provides example tests illustrating FLASH capability; and
Fig. 5 illustratively depicts a method for evaluating a radiation beam for FLASH capability.

### Detailed Description

The term "biological dosimeter" should be understood within the context of this document as a means for evaluating the effects of radiation based on biological processes. Naturally, the interaction between radiation and any biological material involves physical and chemical processes as well which work in concert to achieve any biological effects.

As is described hereafter in greater detail, the biological dosimeters considered herein involve the utilization of only non-mammalian fertilized eggs. Thus, the organisms involved are simple organisms which are routinely used in laboratory research and require much lower standards of ethical oversight approval than, for example, mice, rabbits or monkeys. The specific use of the device and methods described herein is for the evaluation of FLASH-radiotherapy treatment capability of a radiation beam. Thus, the device and methods are to be used in the context of enabling effective treatment of cancer to preserve and prolong the lives of humans and higher order animals.

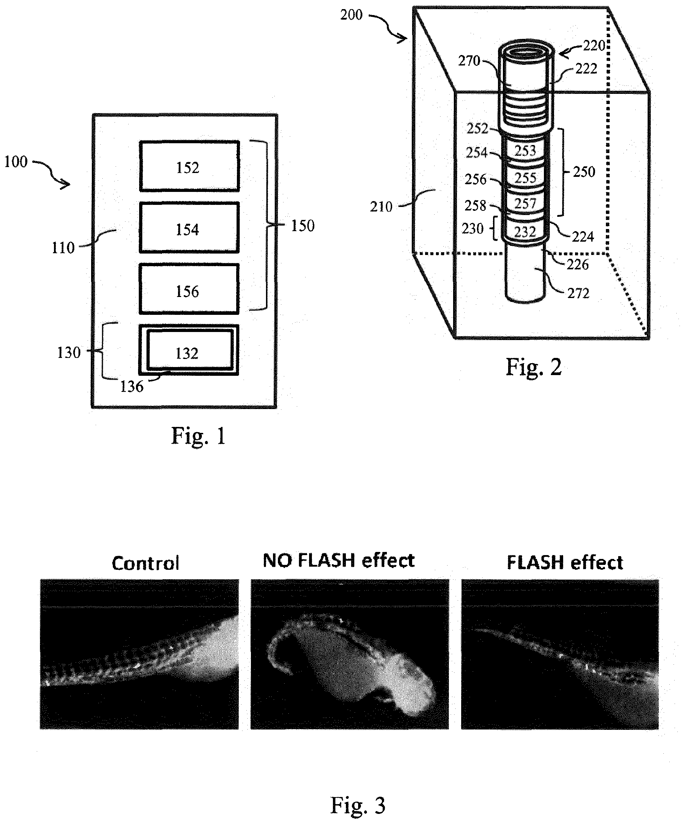

Fig. 1 depicts an illustrative kit 100 for evaluating FLASH capability of a radiation beam. In this example the kit 100 includes a holder 110 within which all other components are mounted. This need not, however, be the case, as the kit 100 may include multiple independent components which are simply packaged and provided together. A holder 110 may be preferred in, for example, clinical settings for ease of transport and/or use. Such a holder 110 may have a first dimension between 2 cm and 10 cm, a second dimension of between 1 cm and 5 cm, and a third dimension between 1 cm and 5 cm. The holder 110 may be shaped as a cuboid. However, the holder 110 should not be limited to said dimensions and is adaptable and arrangeable according to the level of beam energy and characteristics. For instance, a narrow radiation beam may require a smaller, more compact holder 110 or a holder 110 with rounded sides.

The test chamber may, in some configurations comprise a substrate, wherein a cavity is formed within the translucent substrate and the physical dosimeter 150 and the biological dosimeter 130 are positioned within the cavity. The substrate may be translucent, or at least partially transmit wavelengths suitable for radiotherapy, for example, the substrate may be formed of Plexiglas®, glass, translucent plastic and/or transluscent polymer. The substrate may be substantially transparent to wavelengths suitable for radiotherapy. The substrate may promote functioning as a phantom by helping to ensure ionic equilibrium and homogeneous irradiation of the physical and biological dosimeters 150, 130.

A cavity within the substrate may be formed as a bore hole or a type of through hole within the substrate and may occupy less than 50% of the volume of the holder 110, preferably less than 40% of the volume of the holder 110, and more preferably less than 30% of the volume of the holder. In such a configuration, the biological dosimeter 130 and the physical dosimeter 150 may be positioned substantially centrally within the substrate with regard to at least one of the dimensions of the holder 110.

The kit 100 includes a biological dosimeter 130 which includes a test chamber 136 forming a holding area for a number of fertilized non-mammalian eggs (not shown). The test chamber 136 is configured to allow a beam of radiation to impinge upon the eggs with a minimal amount of absorption and/or reflection of the beam due to the test chamber 136. The test chamber 136 may be a cuboid container, however, many different sizes and shapes of test chambers 136 are contemplated. Specifically, a cylindrical test chamber 136 may have certain beam testing benefits as radiation beams may present a circular profile.

The biological dosimeter 130 comprises a number of fertilized eggs which can be selected from non-mammalian animals. As the development of certain embryonic organisms has been studied in more detail, such fertilized eggs may be easier to quantify and evaluate for reproducibility than other organism types. In particular, zebrafish eggs, xenopus eggs, and drosophila eggs are often used as model developmental systems having been studied with particular detail and may be suitable for use in the biological dosimeter 130. One particular configuration of the biological dosimeter includes zebrafish eggs which are provided between 0.5 hours and 36 hours after fertilization, preferably between 1 hour and 30 hours after fertilization, and more preferably between 4 hours and 24 hours after fertilization. It is during this time window after fertilization that zebrafish eggs undergo rapid development and are particularly sensitive to the disruptive effects of radiation. Necessarily, if a different fertilized egg type is used, the developmental window most useful for radiation beam evaluation may be different. In this context zebrafish may provide a more easily evaluated model for radiation evaluation, as zebrafish have been previously employed in the evaluation of, for instance, radiation protectors, such as in McAleer, Mary Frances M.D. et al., "Novel Use of Zebrafish as a Vertebrate Model to Screen Radiation Protectors and Sensors" International Journal of Radiation Oncology: Biology Physics, Volume 61, Issue 1, 10 - 13.

In order to provide a sufficient evaluation of radiation beam FLASH capability, a number of fertilized eggs must be used. Generally this includes at least 5 eggs, preferably at least 10 eggs, more preferably at least 15 eggs, and most preferably at least 20 eggs. In principal there is no upper limit to the number of fertilized eggs used in the biological dosimeter 130, however, practical limits of space and expense may be considered in this regard.

As the fertilized eggs must be kept alive and enabled to develop as normally as possible, the test chamber 136 may also comprise a medium being adapted to promote survival of the fertilized eggs. Depending on the requirements of the radiation beam and the number of eggs, the medium may have a volume of at least 200 µL, preferably at least 500 µL, and more preferably at least 1 mL. It may be preferable to have the test chamber 136 be completely sealed, such that no gases or fluids are exchanged with the outside environment. However, in some circumstances it may be preferable that the test chamber 136 be gas permeable and water impermeable so that oxygen may be replenished and waste gases expelled from the test chamber 136. In other configurations, the holder 110 itself may enable the exchange of gases with the test chamber 136.

One configuration in which the biological dosimeter 130 includes zebrafish eggs may then require a medium within the test chamber 136 comprising water, and more specifically, may closely resemble the medium of ocean water, which is the natural environment for zebrafish. This medium may include an approximate salinity of at least 30 parts per thousand, preferably at least 32 parts per thousand. One commercial example of a suitable medium for zebrafish is Instant Ocean Salt®. The medium may comprise between 10 mg/L and 100 mg/L of Instant Ocean Salt®, preferably between 40 mg/L and 80 mg/L Instant Ocean Salt®, more preferably between 50 mg/L and 70 mg/L Instant Ocean Salt®, most preferably 60 mg/L Instant Ocean Salt®.

As shown in Fig. 1, the kit 100 also includes a physical dosimeter 150 in order to provide dosimetric radiation information of the beam radiation. The physical dosimeter 150 includes at least one type of radiation dosimeter 152, but may include multiple different radiation dosimeters 152, 154, 156 for measuring beam radiation.

In some configurations of the kit 100, the physical dosimeter 150 includes at least one passive dosimeter, i.e. a dosimeter which measures total absorbed radiation and does not actively and continuously report radiation particle counts. It is preferred that the physical dosimeter 150 includes at least one of an alanine dosimeter, Electron paramagnetic resonance (EPR) dosimeter, a Gafchromic film dosimeter, a thermoluminescent dosimeter, and a radiochromic film dosimeter. These physical dosimeters are validated to be dose rate independent (see, for example, M. Jaccard et al., High dose-per-pulse electron beam dosimetry: Usability and dose-rate independence of EBT3 Gafchromic films. Med Phys 44, 725-735 (2017)).

Gafchromic film contains a dye that changes color due to exposure to ionizing radiation and provides the advantage of being self-developing and being generally insensitive to visible light. Alanine dosimeters utilize the amino acid alanine, which due to exposure to ionizing radiation produces unpaired electrons. Alanine dosimeters provide the advantages of being resistant to the influence of temperature, humidity and dose rate and provide a wide measuring range. Thermoluminescent detectors contain a thermoluminescent crystalline material such as, for example, calcium fluoride or lithium fluoride. When exposed to radiation, some of that energy is stored within the crystalline lattice. When heated, light is emitted from the thermoluminescent material having an intensity related to the magnitude of the radiation exposure. Thermoluminescent detectors provide a wide measurement range including low doses and an easy readout mechanism. Another widely used dosimeter is an electron paramagnetic resonance detector (EPR) which provides a good compromise between sample size and sensitivity.

A kit 100 is also envisioned that comprises three different types of radiation dosimeters 152, 154, 156, such as those already mentioned. Such a configuration may be advantageous as said dosimeter types function using different physical mechanisms and have different optimal radiation measurement ranges and saturation thresholds. Thus, multiple physical dosimeters 152, 154, 156 of different physical types helps to ensure accurate and reliable absorbed radiation dose measurements. Additionally, it may be beneficial to provide two or more of each type of dosimeter, such that radiation measurements may be checked against each other. In one possible configuration the physical dosimeter 150 may comprise two or more alanine dosimeters and two or more Gafchromic film dosimeters, preferably positioned in an alternating arrangement.

In Fig. 2 an example configuration of the kit 200 is illustrated. In this example the holder 210 may be a cuboid block of Plexiglas® having dimensions as previously described. A cavity 220 is formed within the central area of the holder 210, preferably by drilling, but may also be formed through alternate means. The cavity 220 can comprise three parts, including a first alignment cavity 222, a test cavity 224, and a second alignment cavity 226, wherein the biological dosimeter 230 and the physical dosimeter 250 are located within the test cavity 224 part of the cavity 220. The first and second alignment cavities 222, 226 may comprise alignment screws 270, and 272, respectively. Each screw 270, 272 engages with the walls of the cavity 220, for example through a corresponding wall thread, in order to firmly maintain the screw 270, 272 in place within the holder 210. The screws 270, 272 help to ensure a central and secure placement of the biological dosimeter 230 and the physical dosimeter 250. The first alignment cavity 222 may have a diameter of 1.0 cm and a length of 2.0 cm. The second alignment cavity 226 may have either the same measurements or the same length with a slightly smaller diameter of 0.6 cm. The second alignment cavity 226 may be narrowed with respect to the first alignment cavity 222 and the test cavity 224, as this assists in positioning the biological dosimeter 230 and the physical dosimeter 250.

In this configuration the biological dosimeter 230 comprises a test chamber 236 wherein an amount of survival medium as previously described and the fertilized eggs are contained. The physical dosimeter 250 in this configuration includes three alanine radiation detectors (in pellet form) 253, 255, 257 as well as four Gafchromic film detectors 252, 254, 256, 258. In this configuration the Gafchromic film detectors 252, 254, 256, 258 and the alanine radiation detectors 253, 255, 257 occupy the test cavity 224 in an alternating arrangement. Thus, assembly of the kit 200 involves production of the holder 210 with cavity 220, positioning of the second screw 272 within the alignment cavity 226, positioning of the biological dosimeter 230, positioning the physical dosimeter 250, then positioning of the first screw 270 within the first alignment cavity 222. As such, multiple redundant physical dosimeters allows for a very accurate determination of the absorbed radiation dose.

Fig. 3 provides an example of the effects of FLASH and non-FLASH radiation on one type of biological dosimeter 130, namely zebrafish eggs. In the left-most image a zebrafish has been allowed to develop normally (control) and clearly exhibits a head with a long, straight tail. In contrast, the center image depicts a zebrafish which has been exposed to a non-FLASH dose of radiation during development. As can be observed, the zebrafish now exhibits a curved and markedly shorter tail when compared with the control zebrafish. In the right-most image a zebrafish has been exposed to a high dose of radiation from a radiation beam configured to provide the FLASH effect. As can be taken therefrom, the zebrafish has developed essentially normally after experiencing FLASH type radiation, having a long, straight tale which is generally comparable with that of the control fish.

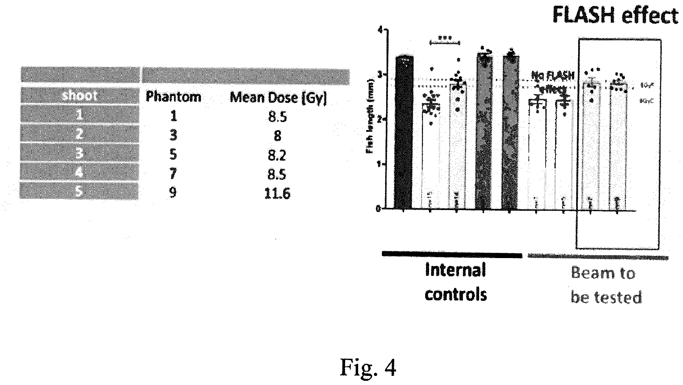

Fig. 4 provides some preliminary data taken from zebrafish which have been tested under various conditions, including controls (left-most five bars) in contrast with a high dose radiation beam (right-most four bars). The zebrafish length has been measured after incubation and as can be observed, zebrafish which did not experience the FLASH effect from a certain radiation beam configuration were significantly shorter than the zebrafish which did experience a radiation beam imparting the FLASH effect. The data from this study was taken over 5 different radiation exposures with a variety of mean absorbed radiation doses measured in Gray (Gy), as is shown in the left-side table.

Any absorbed radiation doses measured by the physical detectors are measured in Gray, and represents an absorbed dose to water value which is traceable to international standards and comprises an inherent related uncertainty.

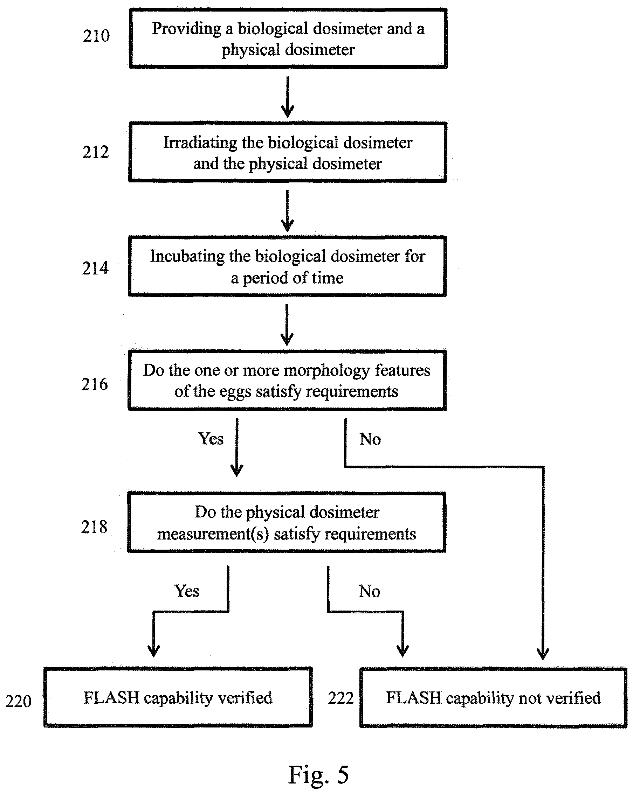

Fig. 5 provides a flow chart depicting the method steps for evaluating the FLASH capability of a radiation beam. The method may be performed using the kit for evaluating FLASH capability in any of the configurations as described above

Initially in step 210, a user, for example a clinician, a medical physicist, or a technician is provided with a kit 100 comprising the biological dosimeter 130 and the physical dosimeter 150.

Providing the kit 100 may involve assembling the kit at a remote laboratory or manufacturing facility and delivering the kit by courier or through the mail to a user. Alternatively, said kit 100 may also be assembled on site. Generally, providing the kit may comprise the steps of placing one or more male/female pairs of breeding organisms into breeding tanks. After fertilization the eggs can then be harvested and each egg checked for normal morphology and confirmed fertilization. The fertilized eggs can then be placed within the test chamber 136 of the kit 100. The physical dosimeter 150 is then assembled together with the biological dosimeter 130 and then the kit 100 may be provided to the user. Depending on the organism used, it may be important to note the exact time of fertilization of the eggs and thus the developmental age of the embryos and make this information available to the user and the provider.

As shown in step 212, the biological dosimeter 130 and the physical dosimeter 150 are then placed in the radiotherapy beam path and irradiated using whatever beam configuration is to be tested for FLASH capability. As previously mentioned, depending on the organism the developmental age of the embryos when subject to radiation can be very important. For example, zebrafish embryos provide optimal radiation sensitivity for different total dose amounts at different developmental ages. As an example, for an irradiation dose of between 2 Gy and 10 Gy zebrafish eggs 4 hours post-fertilization may provide optimal sensitivity. Similarly, for a total irradiation dose of between 20 Gy and 35 Gy zebrafish eggs 24 hours post-fertilization may provide optimal sensitivity. For an intermediate irradiation dose between 10 Gy and 20 Gy zebrafish eggs 6 hours post-fertilization may provide optimal sensitivity.

In contrast fertilized xenopus eggs are most sensitive to radiation effects between stages 6 and 8 of embryonic development (roughly 3 hours post-fertilization to 6 hours post-fertilization) for a total radiation dose of between 30 Gy and 50 Gy. Fertilized drosophila eggs in the early pupa stage (roughly 5-6 days post fertilization) are sensitive to a total radiation dose between 100 Gy and 1000 Gy. Drosophila in the first instar larva stage (roughly 12 hours to 36 hours post-fertilization) are sensitive to total radiation doses between 1000 Gy and 1500 Gy. Drosophila in the second instar larva stage (roughly 36 hours post-fertilization to 60 hours post-fertilization) are sensitive to total radiation doses between 1500 Gy and 2000 Gy. The values provided above are, however, only guides for use of biological dosimeter 130 and should not be interpreted to exclude other valuable radiation dose limits and developmental age ranges.

In the next step 214, the biological dosimeter is incubated for a predetermined period of time. Incubation may include maintaining the fertilized eggs at a specific temperature, such as between 20° C and 30° C, or preferably between 25° C and 28° C. Incubation may also include providing the fertilized eggs with sufficient oxygen, fluid medium, and/or other nutrients such that the fertilized eggs are provided with all necessary requirements such that they could continue developing normally. Incubation may last between 1 and 10 days, preferably between 2 and 6 days, and more preferably between 3 and 5 days. It is envisioned that the kit 100 can be transmitted or mailed back to the kit provider who can then perform the incubation and subsequent steps. Alternatively, the user may perform said steps on site.

After incubation, in step 216 the fertilized eggs, i.e. embryos, are evaluated for one or more morphological features of the fertilized eggs. This evaluation involves comparing the one or more morphological features against a predetermined threshold. For example, if the eggs provided are zebrafish eggs, then the length of the embryos can be measured and this measurement statistic is compared against a normal threshold for the length of a zebrafish. If the egg development 140 does not satisfy the threshold, for example, by being substantially statistically different from the control condition, then it is established that FLASH capability has not been provided by the beam configuration as in step 222. However, if the one or more morphology features does satisfy the threshold, for example by being substantially unchanged from the control conditions, then a subsequent evaluation step 218 is initiated.

As with the timing of radiation exposure of the fertilized eggs relative to developmental age, the timing of morphological evaluation may also be important, depending on the organism in use. As a general guide, a few days, such as 2-5 days post fertilization, preferably 3-5 days post fertilization, can provide sufficient time for embryonic development. Before evaluation the development of the organisms can be halted, for example, with the addition of a 4g/L tricaïne solution to the test chamber 136, and then the organisms are fixed, for example, with a PFA 4% solution, such that accurate morphological evaluation is possible without organism movement or degradation.

As previously mentioned, the morphological evaluation employed in step 216 may include measuring the length of the organisms. In some cases measuring the greatest cross-sectional length of the embryos may be advantageous. Length is a particularly good measure when evaluating developed zebrafish, xenopus tadpoles, or drosophila. Other alternative morphological measures may also be evaluated, including the measure of the eyes and the size of the head, such as the diameter or circumference, and/or the degree of curvature of the organism. In some cases the morphological evaluation of the embryos may involve generating a composite measure of two or more morphological measures. An alternative morphological evaluation step may be to simply evaluate what proportion of the embryos has continued to survive. This may be particularly useful when high absorbed radiation doses are to be tested.

A summary morphology statistic is then derived from the measured values. Such a summary morphology statistic may be a mean value, a median value, quartile values, a standard deviation value, etc. The summary morphology statistic is then compared against a threshold. In some cases the threshold may be at most a 20% reduction from a summary morphology statistic derived from non-irradiated fertilized eggs of the same developmental age, preferably at most a 15% reduction, more preferably at most a 10% reduction. In this way, the irradiated embryos can be compared against embryos which have been incubated under control conditions.

In the evaluation step 218 the one or more physical dosimeters are evaluated such that the total absorbed radiation dose to water value traceable to international standards is reported. This may be a single value or a summary statistic and will naturally have an associated uncertainty value. If the physical radiation measurement is sufficient to overcome a predetermined threshold, such as being large enough to impart therapeutic value, then FLASH capability of the beam has been verified 220. Naturally, if the measured radiation dose is too low to provide therapeutic value, then FLASH capability has not been verified 222.

A possible addition to the method of FLASH-RT evaluation comprises repeating steps 210 through 218 of the method for a second radiotherapy configuration, preferably wherein the second configuration involves imparting substantially the same total absorbed radiation dose, albeit over a longer timescale. The timescale of the second configuration may be at least 100 times longer, preferably 1000 times longer than the timescale of the first configuration. Notably, longer time scales with high radiation doses may fall into the category of "traditional" radiotherapy treatment. "Traditional" or "conventional" radiation therapy may take place over a timescale of minutes, i.e. over at least 10 seconds, preferably at least 100 seconds, more preferably at least a minute. Whereas FLASH-RT may take place over a timescale of microseconds, i.e. over a timescale of less than 1 second, preferably less than 100 milliseconds, more preferably less than 1 millisecond. Thus, for such a method the predetermined threshold is that the summary statistic derived from the first configuration is at least a 10% increase from a summary morphology statistic derived from the second configuration of the radiation beam, preferably at least a 15% increase, more preferably at least a 20% increase. As traditional radiation doses are known to result in more damage to healthy tissues, thus leading to developmental abnormalities, the protective effects of FLASH-RT can be exhibited by a change in said morphological parameters as demonstrated in Fig. 3. An assay comparing both a traditional radiation dose and a FLASH-RT dose may be advantageous for radiotherapy providers who perform both types of the radiotherapy treatment.

While the invention has been illustrated and described in detail in the drawings and foregoing description, such illustration and description are to be considered illustrative or exemplary and non-restrictive; the invention is thus not limited to the disclosed embodiments. Variations to the disclosed embodiments can be understood and effected by those skilled in the art and practicing the described invention, from a study of the drawings, the disclosure, and the appended claims. In the aspects and claims, the word "comprising" does not exclude other elements or steps, and the indefinite article "a" or "an" does not exclude a plurality and may mean "at least one".

The following are preferred aspects of the invention:
1. A kit for evaluating FLASH capability of a radiation beam, the kit including:
   a biological dosimeter comprising a test chamber containing fertilized non-mammalian eggs, wherein the test chamber is further configured to receive a beam of radiation; and
   a physical dosimeter comprising at least one type of a radiation dosimeter.
2. The kit of aspect 1, wherein the fertilized eggs are selected from zebrafish eggs, xenopus eggs, and/or drosophila eggs.
3. The kit of aspect 1, wherein the eggs are zebrafish eggs between 0.5 hours and 36 hours after fertilization, preferably between 1 hour and 30 hours after fertilization, more preferably between 4 hours and 24 hours after fertilization.
4. The kit of any one of the previous aspects, wherein the fertilized eggs comprise at least 5 eggs, preferably at least 10 eggs, more preferably at least 15 eggs, most preferably at least 20 eggs.
5. The kit of any one of the previous aspects, wherein the at least one radiation dosimeter is a passive dose-rate independent dosimeter, preferably wherein the physical dosimeter comprises at least one of the following dosimeter types: an alanine detector, a Gafchromic film, and a thermoluminescent detector.
6. The kit of any one of the previous aspects, wherein the physical dosimeter comprises three different types of radiation dosimeters, preferably wherein the physical dosimeter comprises at least one of an alanine detector, a Gafchromic film, and a thermoluminescent detector.
7. The kit of any one of the previous aspects, wherein the kit comprises a test chamber within which both the biological dosimeter and the physical dosimeter are located.
8. The kit of aspect 7, wherein the test chamber has a first dimension of between 2 and 10 cm, a second dimension of between 1 and 5 cm, and a third dimension of between 1 and 5 cm adaptable to beam energy and characteristics.
9. The kit of aspect 7 or aspect 8, wherein the test chamber comprises a translucent substrate with a central cavity, wherein the physical dosimeter and the biological dosimeter are located within the cavity.
10. The kit of any one of the previous aspects, wherein the biological dosimeter further comprises a medium within the test chamber, the medium being adapted to promote survival of the fertilized eggs, preferably wherein the medium has a volume of at least 200 µL, more preferably at least 500 µL, and even more preferably at least 1 mL.
11. The kit of aspect 10, wherein the medium comprises water comprising between 10 mg/L and 100 mg/L of instant ocean salt, preferably between 40 mg/L and 80 mg/L ocean salt, more preferably between 50 mg/L and 70 mg/L instant ocean salt, most preferably 60 mg/L Instant ocean salt.
12. A method for evaluating FLASH capability of a radiation beam, the method comprising the steps of:
   (i) providing a biological dosimeter comprising a test chamber and fertilized non-mammalian eggs positioned within the test chamber;
   (ii) providing a physical dosimeter comprising at least one type of radiation dosimeter;
   (iii) irradiating the biological dosimeter with a radiation beam having a first configuration;
   (iv) irradiating the physical dosimeter with a radiation beam having the first configuration;
   (v) incubating the irradiated eggs for a predetermined time;
   (vi) evaluating one or more morphology features of the fertilized eggs after incubation;
   (vii) evaluating the irradiated physical dosimeter; and
   (viii) classifying the FLASH capability of the radiation beam based at least in part on the evaluation of the irradiated physical dosimeter and the morphology evaluation.
13. The method of aspect 12, wherein evaluating the morphology comprises generating a summary morphology statistic derived from measurements made of each incubated fertilized egg, preferably by measuring a greatest cross-sectional length of each of the embryos and/or survival evaluation of the eggs.
14. The method of aspect 12, wherein evaluating the morphology comprises generating a summary morphology statistic derived from measuring a length of the incubated fertilized eggs, measuring a diameter of the eyes, measuring a size of the head, determining the curvature of the fish, and/or evaluating the survival of the eggs.
15. The method of any one of aspects 12-14, wherein evaluating the physical dosimeter radiation measurement comprises reading at least one radiation dosimeter and deriving an absorbed dose to water value.
16. The method of any one of aspects 12-15, wherein evaluating the physical dosimeter radiation measurement comprises reading at least two dosimeters, preferably at least three dosimeters, and generating an absorbed dose to water value, wherein each dosimeter operates based on a different physical radiation measurement mechanism.
17. The method of any one of aspects 12-16, wherein classifying the FLASH capability of the first configuration based on the evaluation of the irradiated physical dosimeter and the morphology evaluation comprises
   determining whether the summary morphology statistic meets a predetermined threshold; and
   determining whether the summary physical dosimeter statistic meets a predetermined threshold.
18. The method of aspect 17, wherein the predetermined threshold for the summary morphology statistic is at most a 20% reduction from a summary morphology statistic derived from non-irradiated fertilized eggs of the same developmental age, preferably at most a 15% reduction, more preferably at most a 10% reduction.
19. The method of any one of aspects 17-18, the method further comprising repeating the steps i) through vii) using a second configuration of the radiation beam, and wherein classifying the FLASH capability of the radiation beam comprises the evaluation of the irradiated physical dosimeter and performing the morphology evaluation of the first and second configurations.
20. The method of aspect 18, wherein the second configuration of the radiation beam is configured to provide the same radiation dose over a timescale at least 100 times longer than the first configuration, and wherein the predetermined threshold for the summary morphology statistic is at least a 10% increase from a summary morphology statistic derived from the second configuration of the radiation beam, preferably at least a 15% increase, more preferably at least a 20% increase.
21. The method of any one of aspects 17-20, wherein the first predetermined threshold for the value of the absorbed dose to water measured in Gy, which when applied to fertilized eggs over a timescale at least 100 times longer than a total duration of the first configuration results in shortening of the average fertilized egg length after incubation relative to normal fertilized egg development by at least 3%, preferably at least 5%, more preferably at least 7%.
22. The method of any one of aspects 12-21, wherein incubation comprises maintaining the irradiated eggs at a temperature between 20°C and 35°C, preferably between 25°C and 30°C, more preferably between 25°C and 29°C, most preferably at 28°C.
23. The method of any one of aspects 12-22, wherein the predetermined time for incubation is between 1 and 10 days, preferably between 2 and 5 days, more preferably between 3 and 5 days.
24. The method of any one of aspects 12-23, comprising the further step of stopping the development of the fertilized eggs after incubation, preferably by immersing the fertilized eggs in a tricaine solution; and/or fixing the fertilized eggs, preferably by immersing them in a solution comprising of PFA and PBS.
25. The method of any one of aspects 12-24, wherein steps iii) and iv) are performed simultaneously.
26. The method of any one of aspects 12-25, wherein each dosimeter is chosen from the list comprising: an alanine dosimeter, Electron paramagnetic resonance (EPR) dosimeter, a Gafchromic film dosimeter, a thermoluminescent dosimeter, and a radiochromic film dosimeter.
27. The method of any one of aspects 12-26, wherein providing a biological dosimeter comprises
   breeding non-mammalian eggs;
   selecting the non-mammalian eggs that are fertilized; and
   positioning the fertilized non-mammalian eggs into the test chamber.

## Claims

1. A kit for evaluating FLASH capability of a radiation beam, the kit including:
a biological dosimeter comprising a test chamber containing fertilized non-mammalian eggs, wherein the test chamber is further configured to receive a beam of radiation; and
a physical dosimeter comprising at least one type of a radiation dosimeter.

2. The kit of claim 1, wherein the fertilized eggs are selected from zebrafish eggs, xenopus eggs, and/or drosophila eggs.

3. The kit of claim 1, wherein the eggs are zebrafish eggs between 0.5 hours and 36 hours after fertilization, preferably between 1 hour and 30 hours after fertilization, more preferably between 4 hours and 24 hours after fertilization.

4. The kit of any one of the previous claims, wherein the fertilized eggs comprise at least 5 eggs, preferably at least 10 eggs, more preferably at least 15 eggs, most preferably at least 20 eggs.

5. The kit of any one of the previous claims, wherein the at least one radiation dosimeter is a passive dosimeter, preferably wherein the physical dosimeter comprises at least one of the following dose-rate independent dosimeter types: an alanine detector, a Gafchromic film, and a thermoluminescent detector; more preferably wherein the physical dosimeter comprises three different types of radiation dosimeters, preferably wherein the physical dosimeter comprises at least one of an alanine detector, a Gafchromic film, and a thermoluminescent detector.

6. The kit of any one of the previous claims, wherein the kit comprises a test chamber within which both the biological dosimeter and the physical dosimeter are located.

7. The kit of any one of the previous claims, wherein the biological dosimeter further comprises a medium within the test chamber, the medium being adapted to promote survival of the fertilized eggs, preferably wherein the medium has a volume of at least 200 µL, more preferably at least 500 µL, and even more preferably at least 1 mL.

8. A method for evaluating FLASH capability of a radiation beam, the method comprising the steps of:
(i) providing a biological dosimeter comprising a test chamber and fertilized non-mammalian eggs positioned within the test chamber;
(ii) providing a physical dosimeter comprising at least one type of radiation dosimeter;
(iii) irradiating the biological dosimeter with a radiation beam having a first configuration;
(iv) irradiating the physical dosimeter with a radiation beam having the first configuration;
(v) incubating the irradiated eggs for a predetermined time;
(vi) evaluating one or more morphology features of the fertilized eggs after incubation;
(vii) evaluating the irradiated physical dosimeter; and
(viii) classifying the FLASH capability of the radiation beam based at least in part on the evaluation of the irradiated physical dosimeter and the morphology evaluation.

9. The method of claim 8, wherein evaluating the morphology comprises generating a summary morphology statistic derived from measuring a length of the incubated fertilized eggs, measuring a diameter of the eyes, measuring a size of the head, determining the curvature of the fish, and/or evaluating the survival of the eggs.

10. The method of claim 8 or claim 9, wherein evaluating the physical dosimeter radiation measurement comprises reading at least one radiation dosimeter and deriving an absorbed dose to water value.

11. The method of any one of claims 8-10, wherein evaluating the physical dosimeter radiation measurement comprises reading at least two dosimeters, preferably at least three dosimeters, and generating an absorbed dose to water value, wherein each dosimeter operates based on a different physical radiation measurement mechanism.

12. The method of any one of claims 8-11, wherein classifying the FLASH capability of the first configuration based on the evaluation of the irradiated physical dosimeter and the morphology evaluation comprises
determining whether the summary morphology statistic meets a predetermined threshold; and
determining whether the summary physical dosimeter statistic meets a predetermined threshold.

13. The method of claim 12, wherein the predetermined threshold for the summary morphology statistic is at most a 20% reduction from a summary morphology statistic derived from non-irradiated fertilized eggs of the same developmental age, preferably at most a 15% reduction, more preferably at most a 10% reduction.

14. The method of any one of claims 12-13, the method further comprising repeating the steps i) through vii) using a second configuration of the radiation beam, and wherein classifying the FLASH capability of the radiation beam comprises the evaluation of the irradiated physical dosimeter and performing the morphology evaluation of the first and second configurations, preferably wherein the second configuration of the radiation beam is configured to provide the same radiation dose over a timescale at least 100 times longer than the first configuration, and wherein the predetermined threshold for the summary morphology statistic is at least a 10% increase from a summary morphology statistic derived from the second configuration of the radiation beam, preferably at least a 15% increase, more preferably at least a 20% increase.

15. The method of any one of claims 12-14, wherein steps iii) and iv) are performed simultaneously.
